# EUROPÄISCHE PATENTSCHRIFT

(11) **EP 0 426 795 B1**
(45) Veröffentlichungstag und Bekanntmachung des Hinweises auf die Patenterteilung: **26.04.1995**
(21) Anmeldenummer: 90906835.5
(22) Anmeldetag: 10.05.1990
(51) Int. Cl.: A61B 3/14, A61B 3/135

(54) **PHOTOZUSATZ UND BLENDENANORDNUNG FÜR EIN BINOKULARES MIKROSKOP**
PHOTOGRAPHIC ATTACHMENT AND SHUTTER DEVICE FOR A BINOCULAR MICROSCOPE
ACCESSOIRE PHOTOGRAPHIQUE ET DISPOSITIF A DIAPHRAGME POUR MICROSCOPE BINOCULAIRE

(30) Priorität: 11.05.1989 EP 89810350
(43) Veröffentlichungstag der Anmeldung: 15.05.1991
(73) Patentinhaber: HAAG-STREIT AG Werkstätten für Präzisionsmechanik, CH-3098 Köniz (CH)
(72) Erfinder: PAPRITZ, Franz, CH-3145 Niederscherli (CH); WIDMER, Hansruedi, CH-3145 Niederscherli (CH)
(74) Vertreter: Steiner, Martin
(86) Internationale Anmeldenummer: CH9000126
(87) Internationale Veröffentlichungsnummer: WO9013255

(56) Entgegenhaltungen:
- FR-A- 1 129 603
- US-A- 3 533 342
- US-A- 4 265 518

## Beschreibung

Die vorliegende Erfindung betrifft einen Photozusatz für ein Mikroskop, insbesondere ein ophtalmologisches Mikroskop zur Beobachtung eines mit Hilfe einer Spaltlampe beleuchteten, Auges gemäss dem Oberbegriff des Anspruchs 1. Eine derartige Anordnung, allerdings nicht im Zusammenhang mit einem besonderen Zusatz zu einem bestehenden Mikroskop sondern in ein Spezialinstrument integriert, ist bekannt aus der US-A-4 265 518. Dabei dient die verstellbare Blende der Anpassung an die eingestellte Vergrösserung, d.h., die Blende wird immer nur verstellt wenn auch die Vergrösserung geändert wird. Im übrigen bleibt die einmal eingestellt Blende bzw. Vergrösserung unverändert wenn von Beobachtung zu Aufnahme übergegangen wird.

Es zeigt sich, dass diese Anordnung nicht befriedigt, weil zur optimalen Beobachtung eine andere Blendeneinstellung erforderlich ist als für eine optimale photographische Aufnahme.

Es ist zwar bekannt, in Spiegelreflexkameras mit der Spiegelverschwenkung auch die Blende aus der voll offenen Stellung in eine vorgewählte Stellung für die photographische Aufnahme zu bringen, aber die für Spiegelreflexkameras geeignete Anordnung ist nicht für einen Photozusatz an einem Mikroskop anwendbar.

Der Erfindung liegt daher die Aufgabe zugrunde, einen Photozusatz an einem Mikroskop zu schaffen, der allen Anforderungen für eine optimale photographische Aufnahme gerecht wird. Die Lösung dieser Aufgabe ist im Kennzeichen des Anspruchs 1 umschrieben. Diese Gesamtkonzeption gestattet es einerseits, die mechanisch bewegten Teile, nämlich den Spiegel und die Blende(n), örtlich nahe beieinander in einem gemeinsamen, in das Mikroskop einfügbaren Gehäuse unterzubringen. Andererseits liegt zwischen der bzw. den Blende(n) und der Kamera ein verhältnismässig grosser Abstand, indem bei der Aufnahme der Strahlengang durch die Blende(n) über den Spiegel und durch den Phototubus verläuft, dies im Gegensatz zu den bekannten Anordnungen wo der Beobachtungsstrahlengang über den Spiegel verläuft. Es werden damit Abschattungen, wie sie bei bekannten Geräten bei bestimmten Vergrösserungen und Blendenstellungen vorkommen, vermieden.

Die Erfindung wird nun anhand eines in der Zeichnung dargestellten Ausführungsbeispiels näher erläutert.
- Fig. 1: zeigt eine Spaltlampe mit dem erfindungsgemässen Photozusatz,
- Fig. 2: zeigt einen Teil der Betätigungsvorrichtung in grösserem Masstab und teilweise geschnitten,
- Fig. 3: zeigt einen Schnitt durch einen Gehäuseteil mit dem Spiegel und der Blende in Ruhestellung,
- Fig. 4: zeigt einen etwas vereinfachten Schnitt gemäss Figur 3 mit dem Spiegel und der Blende in Arbeitsstellung,
- Fig. 5: zeigt einen Teilschnitt durch ein Betätigungsgestänge,
- Fig. 6: zeigt einen Schnitt nach Linie VI/VI in Figur 3,
- Fig. 7: zeigt einen Schnitt nach Linie VII-VII in Figur 3,
- Fig. 8: zeigt die Blende bei grösster Blendenöffnung,
- Fig. 9: zeigt die Blende bei kleinster Blendenöffnung,
- Fig. 10: ist eine Teilansicht einer Ausführungsvariante und
- Fig. 11: zeigt einen Teilschnitt der Ausführungsvariante.

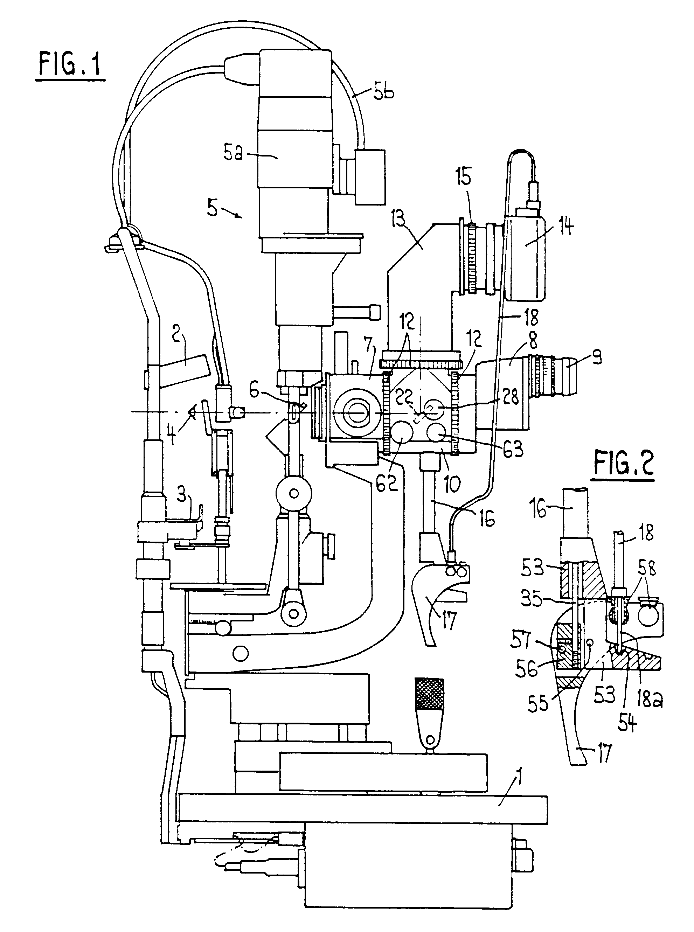

Figur 1 zeigt eine Spaltlampe die mit dem erfindungsgemässen Photozusatz ausgerüstet ist. Diese Spaltlampe ist im Prospekt "die neue Haag-Streit Spaltlampe 900 BQ" der Firma Haag-Streit AG beschrieben, und es werden nur einige im Zusammenhang mit der Erfindung interessierende Teile dieser Spaltlampe erwähnt. Die Spaltlampe ist auf einer Basis 1 montiert und weist eine Stirnstütze 2 und eine Kinnstütze 3 für den Kopf eines Patienten auf, von welchem in Figur 1 nur schematisch das Auge 4 angedeutet ist. Zur Beobachtung des Auges ist eine Spaltlampenbeleuchtung 5 mit Blitzleuchte 5a vorgesehen, welche über einen geneigten Spiegel 6 und eventuelle in Figur 1 angedeutete Zusatzeinrichtungen das Auge 4 zu beleuchten gestattet. Zur Beobachtung des Auges ist ein binokulares Mikroskop vorgesehen, das einen Vergrösserungswechsler 7 und einen Okulartubus 8 mit den Okularen 9 aufweist. Zwischen den Vergrösserungswechsler 7 und den Okulartubus 8 ist ein Gehäuse 10 des erfindungsgemässen Photozusatzes eingesetzt und mittels Fixierringen 12 lösbar befestigt. Auf das Gehäuse 10 ist ein Objektivtubus oder Phototubus 13 ebenfalls mittels Fixierring 12 lösbar aufgesetzt, mit welchem eine Kleinbildkamera 14 mittels eines Fixierringes 15 lösbar verbunden ist. An der Unterseite des Gehäuses 10 ist ein Rohr 16 befestigt in dem sich ein später beschriebenes Betätigungsgestänge befindet. Dieses Betätigungsgestänge ist gekoppelt mit einem Betätigungshebel 17, welcher über ein Kabel 18 auch der Auslösung der Kamera 14 dient. Bei Ruhestellung des Photozusatzes kann das Auge 4 durch das Mikroskop unbehindert betrachtet werden. Während der Beobachtung kann kurzzeitig durch Betätigung des Auslösehebels 17 der Strahlengang des Mikroskops durch den Phototubus 13 zur Kamera 14 ausgelenkt und durch Auslösen der Kamera 14 eine Aufnahme des Auges 4 gemacht werden. Der Aufbau der diese Bedienung ermöglicht, ist im folgenden im einzelnen beschrieben.

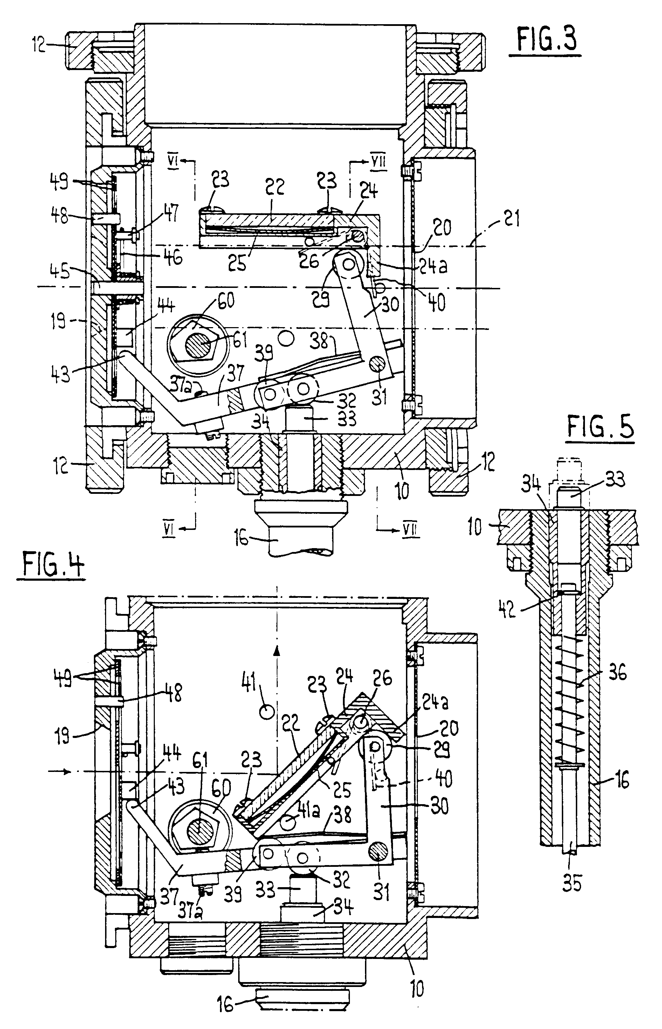

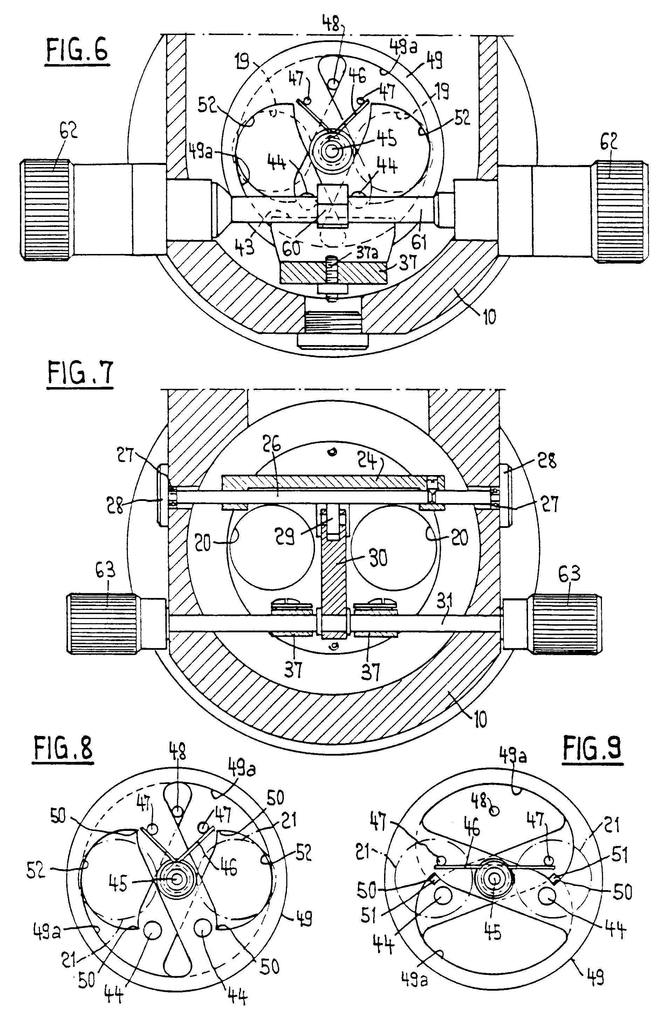

Das Gehäuse 10 weist Eintrittsöffnungen 19 und Austrittsöffnungen 20 für die parallelen Beobachtungsstrahlenbündel 21 des binokularen Mikroskops auf. Im Gehäuse 10 befindet sich ein Spiegel 22 der mittels Schrauben 23 in einer Fassung 24 fein einstellbar ist, indem eine Blattfeder 25 den Spiegel 22 gegen die Schrauben 23 drückt und damit die Lage des Spiegels in der Fassung 24 bestimmt. Die Fassung 24 mit dem Spiegel 22 ist auf einer Schwenkachse 26 befestigt welche mittels Kugellager 27 (Figur 7) in den Gehäusewänden gelagert ist. Die Lagerbohrungen sind mittels Kappen 28 abgedeckt, die in nicht näher dargestellter Weise an der Aussenseite der Gehäusewandung befestigt sind. Bei der in Figuren 3 und 7 dargestellten horizontalen Ruhestellung des Spiegels 22 und seiner Fassung 24 können die Beobachtungsstrahlenbündel 21 unbehindert durchtreten, was insbesondere aus Figur 7 klar ersichtlich ist. Es ist daher eine gute Beobachtung des Auges 4 möglich, indem alles in das Objektiv des Mikroskops eintretende Licht für die Beobachtung verfügbar ist.

Der Spiegel 22 bzw. seine Fassung 24 ist durch eine Schenkelfeder 40 in der in den Figuren 3 und 7 dargestellten Ruhelage an einem Anschlagstift 41 (Fig. 4) gehalten. Unter ihn greift eine Stützrolle 29 die am oberen Ende eines Winkelhebels 30 drehbar gelagert ist. Der Winkelhebel 30 ist schwenkbar um eine Achse 31. Im unteren Arm des Winkelhebels 30 ist eine Rolle 32 gelagert, die auf einen Stift 33 eines Betätigungsgestänges aufliegt. Der Stift 33 sitzt in einer Hülse 34 die im Rohr 16 axial verschiebbar geführt ist. Zwischen einer im Rohr 16 axial verschiebbaren Betätigungsstange 35 und der Hülse 34 wirkt eine Druckfeder 36 welche eine kraftschlüssige Verbindung zwischen der Betätigungsstange 35 und der Hülse 34 bzw. dem Stift 33 herstellt. Es besteht damit auch eine kraftschlüssige, nachgiebige Verbindung zwischen der Betätigungsstange 35 und dem Winkelhebel 30. Auf der Achse 31 ist ein den unteren Arm des Winkelhebels 30 gabelförmig umgreifender Schlepphebel 37 befestigt. Mit dem Schlepphebel 37 ist eine Blattfeder 38 verschraubt, auf die eine Mitnehmerrolle 39 des unteren Armes des Winkelhebels 30 wirkt. Es ist damit eine kraftschlüssige, nachgiebige Verbindung hergestellt zwischen dem Winkelhebel 30 und dem Schlepphebel 37, d.h. wenn der Winkelhebel 30 bei Aufwärtsbewegung der Betätigungsstange 35 bzw. des Stiftes 33 im Uhrzeigersinn aus der in Figur 3 dargestellten Ruhelage verdreht wird, nimmt er den Schlepphebel 37 mit einem begrenzten durch die Feder 38 bestimmten Drehmoment mit. Die beiden Hebel 30 und 37 sind durch ihre Schwerkraft in der dargestellten Ruhelage gehalten, wobei die Rolle 32 auf dem Stift 33 ruht. Die Feder 36 ist hierbei wirkungslos, weil die Hülse 34 gegen einen Segerring 42 am oberen Ende der Betätigungsstange 35 anliegt.

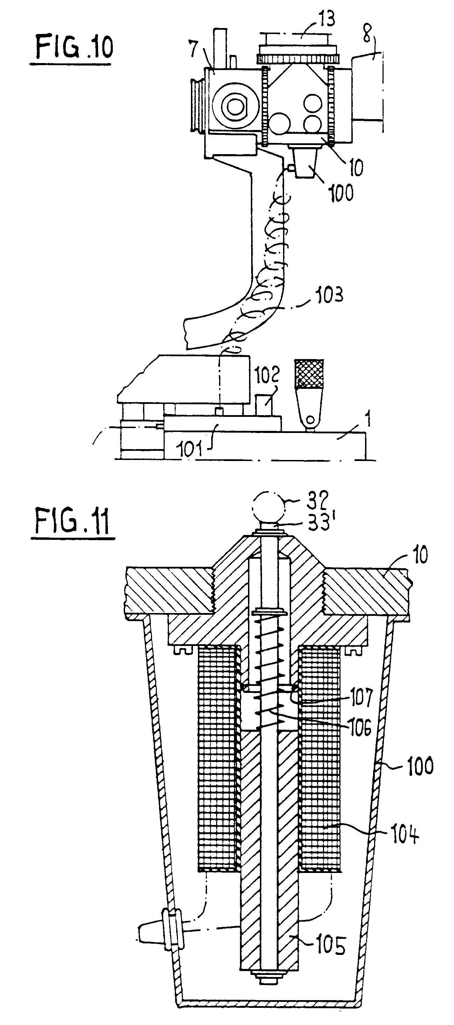

Der Schlepphebel 37 greift mit seiner horizontalen, geraden Kante 43 unter zwei Mitnehmerrollen 44, die an je einer Blendenscheibe 49 gelagert sind. Die beiden Blendenscheiben 49 sind um eine gemeinsame Achse 45 schwenkbar und eine Feder 46 wirkt mit ihren freien Enden auf Stifte 47 die in je eine der Blendenscheiben eingelassen sind, um die Blendenscheiben in ihrer Ruhelage zu halten, bei welcher gemäss Figuren 3 und 8 der Schlepphebel 37 nicht auf die Mitnehmerzapfen 44 wirkt. Diese Ruhestellung der Blendenscheiben ist hierbei durch einen gemeinsamen Anschlagstift 48 bestimmt. Jede Blendenscheibe 49 weist zwei symmetrisch gegenüberliegende etwa halbkreisförmige Fenster 49a auf. Je das eine Ende des gestreckt verlaufenden Teils der Fensterbegrenzung ist auswärts gebogen, womit auswärtsgezogene Fensterecken 50 gebildet sind, die bei der kleinsten Blendenstellung gemäss Figur 9 die Blendenöffnungen 51 bilden. Diese Blendenöffnungen liegen praktisch axialsymmetrisch im Strahlengang 21 der auch in den Figuren 8 und 9 angedeutet ist. Bei Ruhelage gemäss Figuren 3 bzw. 8 sind die Blenden voll offen und bilden Blendenöffnungen 52, die ebenfalls weitgehend axialsymmetrisch zu den Strahlenbündeln 21 liegen. Bei der Ruhelage bzw. Beobachtungsstellung sind also die Blenden voll geöffnet und gestatten praktisch ungehinderten Durchtritt der Beobachtungsstrahlenbündel 21.

Figur 2 zeigt das untere Ende des Rohres 16 und die damit verbundene eigentliche Betätigungsvorrichtung des Photozusatzes. In einem am unteren Ende des Rohres 16 befestigten Träger 53 ist der Betätigungshebel 17 um eine Achse 55 schwenkbar gelagert. Im gabelförmigen oberen Teil dieses Betätigungshebels 17 ist ein Klotz 56 mittels eines Stiftes 57 schwenkbar mit dem Hebel 17 verbunden. Mit diesem Klotz ist das untere Ende der Betätigungsstange 35 verschraubt, und durch Drehen der Stange 35 kann die genaue axiale Position derselben gegenüber dem Betätigungshebel 17 fein eingestellt werden. Mit dem Betätigungshebel 17 sind auch Fassungen 58 zum Ankuppeln mindestens eines Auslösekabels 18 für eine Kamera verbunden. Der Draht 18a des Auslösekabels 18 greift in eine von zwei konischen Vertiefungen 54 des Trägers 53.

Zur Aufnahme eines Bildes des betrachteten Auges 4 braucht der Betrachter lediglich den Betätigungshebel 17 in Figur 1 bzw. 2 nach links zu drücken, womit er im Uhrzeigersinn verschwenkt wird. Die Betätigungsstange 35 wird damit im Rohr 16 nach oben geschoben und nimmt über die Feder 36 die Hülse 34 und den Stift 33 die Rolle 32 des Winkelhebels 30 mit und verschwenkt denselben im Uhrzeigersinn. Damit trifft die Rolle 29 des Winkelhebels 30 auf den nach unten gezogenen Flansch 24a der Fassung 24 des Spiegels 22 auf und verschwenkt diese Fassung mit dem Spiegel um die Achse 26 im Gegenuhrzeigersinn bis in die in Figur 4 dargestellte Schwenklage, in welcher die Fassung des Spiegels gegen einen unteren Anschlagstift 41a anschlägt. Damit wird die Schwenkbewegung des Winkelhebels 30 und die Aufwärtsbewegung des Stiftes 33 und der Hülse 34 begrenzt und eine weitere Aufwärtsbewegung der Betätigungsstange 35 wird durch die Feder 36 aufgenommen, die somit eine kraftschlüssige, nachgiebige Verbindung zwischen der Betätigungsstange 35 bzw. dem Betätigungshebel 17 und dem Winkelhebel 30 darstellt. Der Spiegel 22 befindet sich nun in seiner wirksamen Stellung, in welcher der Strahlengang gemäss Figur 4 nach oben in den Phototubus 13 und zur Kamera 14 umgelenkt wird. Bei der Verschwenkung des Winkelhebels 30 wird über die Feder 38 auch der Schlepphebel 37 mitgenommen, und zwar bis in eine Stellung in welcher eine einstellbare Anschlagschraube 37a gegen einen mehreckigen Anschlagkörper 60 anschlägt. Bei dieser Schwenkbewegung bis zum Anschlag nimmt der Schlepphebel 37 mit seiner Kante 43 die Mitnehmerbolzen 44 der beiden Blendenscheiben 49 mit und verdreht dieselben entgegen der Wirkung der Feder 46 in eine Blendenstellung die durch den einstellbaren Anschlagkörper 60 bestimmt ist. Die Blenden liegen dabei an einer günstigen Stelle zwischen dem Spiegel 22 und dem Vergrösserungswechsler 7 des Mikroskops, also in Richtung des Strahlenganges vor dem Spiegel und insbesondere vor dem Phototubus 13. Es ergeben sich dabei die oben bereits erwähnten günstigen Voraussetzungen für die photographischen Aufnahmen. Die Wege sind so bemessen, dass die Schwenkbewegung des Schlepphebels 37 in jedem Falle durch Anschlag beendet ist, bevor die Schwenkbewegung des Winkelhebels 30 beendet ist. Die Bewegungsdifferenz wird wiederum durch die kraftschlüssige elastische Verbindung mit Hilfe der Blattfeder 38 ausgeglichen. Die Auslösung der Kamera 14 ist so eingestellt, dass sie erst erfolgt, nachdem der Spiegel 22 und der Schlepphebel 37 in ihre jeweiligen Anschlagstellungen geschwenkt worden sind, d.h. wenn der Spiegel 22 die korrekte Stellung gemäss Figur 4 und die Blende die gewollte Oeffnung erreicht hat. Dann wird bei weiterer Bewegung des Betätigungshebels 17 mit angemessener Verzögerung die Kamera 14 ausgelöst. Ueber eine nicht dargestellte Elektronik und ein Kabel 5b wird im richtigen Augenblick auch die Blitzleuchte 5a aktiviert. Wird der Betätigungshebel 17 dann losgelassen, gehen alle bewegten Teile unter Federkraft, bzw. Schwerkraft in ihre Ruhestellungen gemäss Figuren 3, 7 und 8 zurück.

Wie Figur 6 zeigt, ist der Anschlagkörper 60 auf einer Achse 61 befestigt, an deren Enden Betätigungsknöpfe 62 angeordnet sind. Mittels dieser Knöpfe kann die Position des Anschlagkörpers 60 und damit die Blendenöffnung anhand einer nicht dargestellten Skala eingestellt werden. Die Stellungen können durch Rasten bestimmt sein.

Der Schlepphebel 37 ist fest mit der Achse 31 verbunden, an deren äusseren Enden Betätigungsknöpfe 63 befestigt sind. Mittels dieser Knöpfe kann der Schlepphebel während der Beobachtung verschwenkt werden, um die Blende auf die vorgewählte Oeffnung zu schliessen. Es lässt sich damit durch die Okulare überprüfen, ob keine Elemente vor dem Mikroskopobjektiv, z.B. Teile der Spaltbeleuchtung, störende Abschattungen verursachen und den Photostrahlengang beschneiden.

Alle wesentlichen Teile des Photozusatzes sind auswechselbar. So ist das Gehäuse 10 auswechselbar zwischen den Vergrösserungswechsler und den Okulartubus des Mikroskops eingesetzt. Es kann also insbesondere auch ein bestehendes Mikroskop mit dem Photozusatz ohne weiteres ausgerüstet werden. Eine besondere Vereinfachung liegt darin, dass alle Teile des Zusatzes mit dem Gehäuse 10 verbunden sind, also nur dieses Gehäuse in das Mikroskop einzusetzen ist. Auch der Phototubus 13 ist lösbar, sowohl mit dem Gehäuse 10 als auch der Kamera verbunden. Es können also mit dem Gehäuse 10 verschiedene Phototuben und Kameras 14 verwendet werden. Im vorliegenden Falle ist angenommen, es werde im Phototubus 13 und in der Kamera 14 nur ein Strahlengang zur Aufnahme eines Monobildes verwendet. Es kann aber auch eine entsprechende Vorrichtung zur Erstellung von Stereobildern oder aber von Sofortbildern angebaut werden. Entsprechend ist die Betätigungsvorrichtung gemäss Figur 2 auch für verschiedene Gegenbenheiten ausgerüstet, d.h. das Auslösekabel 18 kann wahlweise mit der einen oder anderen Fassung 58 verbunden werden, um einen kürzeren oder längeren Betätigungsweg zu erzielen. Anstelle des mehreckigen Anschlagkörpers 60 könnte eine Kurvenscheibe vorgesehen sein, um eine kontinuierliche Blendeneinstellung zu ermöglichen.

Die Betätigung aller Teile könnte jedoch auch in anderer Weise erfolgen. Es wäre beispielsweise denkbar, die beschriebenen Bewegungen elektrisch über Elektromagnete und dergleichen Stellmittel mit einem Tastendruck auszulösen, wobei elektronisch die richtige zeitliche Reihenfolge der Vorgänge gesteuert werden könnte.

Eine Ausführungsvariante dieser Art ist in den Fig. 10 und 11 dargestellt. In Fig. 10 sind entsprechende Teile gleich bezeichnet wie in den übrigen Figuren. Anstelle des Mechanismus zur Betätigung des Spiegels 22 ist ein Betätigungsmagnet vorgesehen, von welchem in Fig. 10 nur das Gehäuse 100 dargestellt ist. Auf der Basis 1 ist ein Gehäuse 101 mit der elektrischen Ausrüstung befestigt, mit einer Auslösetaste 102. Mit Klemmen des Gehäuses 101 sind ein Kabel 103 zur Erregung des Magneten 100 und mindestens ein weiteres Kabel zur Steuerung der Kamera und der Blitzleuchte verbunden. Fig. 11 zeigt die Spule 104 und den Tauchanker 105 des Elektromagneten. Der Tauchanker 105 sitzt verschiebbar auf einer Stange 33', die der Stange 33 entspricht und deren oberes Ende in der in Fig. 3 und 4 dargestellten Weise auf die Rolle 32 des Schwenkmechanismus für den Spiegel 22 wirkt. Die Bewegung des Tauchankers 105 wird über eine Feder 106 auf die Stange 33' übertragen, um eine kraftschlüssige Betätigung des Spiegels zu erzielen. Ein O-Ring 107 verhindert ein schlagartiges Aufprallen des Tauchmagneten 105 auf seinen Anschlag.

Die Arbeitsweise der Ausführung nach Fig. 10 und 11 entspricht weitgehend der beschriebenen Arbeitsweise der Ausführung nach Fig. 1 - 9 mit dem Unterschied, dass alle Einrichtungsteile elektrisch gesteuert werden. Bei Druck auf die Auslösetaste 102 wird ohne Verzögerung der Elektromagnete 104 erregt und der Spiegel 22 wird in der beschriebenen Weise gekippt. Mit angemessener Verzögerung werden über ein nicht dargestelltes Kabel die Kamera 14 und über das Kabel 5b die Blitzleuchte ausgelöst. Es könnte aber auch ein Kontrollkontakt vorgesehen werden, der betätigt wird, wenn der Spiegel 22 seine für die Aufnahme erforderliche geneigte Lage erreicht hat, und die Auslösung der Kamera und der Blitzleuchte kann durch diesen Kontakt überwacht werden. Als Kontakt kann z. B. der Anschlagstift 41a ausgebildet sein. Die Blitzleuchte kann in bekannter Weise durch die Kamera ausgelöst werden.

## Patentansprüche

1. Photozusatz für ein Mikroskop, insbesondere ein ophtalmologisches Mikroskop zur Beobachtung eines mit Hilfe einer Spaltlampe beleuchteten Auges, mit einer Photokamera (14), mit einem in den Strahlengang bewegbaren, vollreflektierenden Spiegel (22), und mit mindestens einer verstellbaren Blende (44 - 49) an der Lichteintrittsseite vor dem Spiegel (22), gekennzeichnet durch ein vor den Okulartubus (8) des Mikroskops einsetzbares Gehäuse (10), in welchem sich der genannte Spiegel (22) und die Blende(n) befinden und mit welchem Gehäuse ein Phototubus (13) verbunden ist, der die Kamera (14) trägt, derart, dass die jeweilige photographische Aufnahme durch die Blende(n) (44 - 49) über den Spiegel (22) und durch den Phototubus (13) erfolgt, wobei gemeinsame Mittel (35, 105) zur gleichzeitigen Verstellung des Spiegels (22) und der Blende(n) (44 - 49) je aus einer Ruhestellung zur Beobachtung in eine Stellung für die Aufnahme und ein einstellbarer Anschlagkörper (60) zur Vorwahl der Blendenstellung für die Aufnahme vorgesehen sind.

2. Photozusatz nach Anspruch 1, dadurch gekennzeichnet, dass die gemeinsamen Mittel ein Betätigungsorgan (35, 105) sind, welches kraftschlüssig, z.B. über eine Feder (36, 106) auf den Spiegel (22) wirkt, dessen Stellungen durch Anschläge (41, 41a) bestimmt sind.

3. Photozusatz nach Anspruch 2, dadurch gekennzeichnet, dass ein Betätigungshebel (17) verzögert auf den Auslöser der Kamera wirkt.

4. Photozusatz nach einem der Ansprüche 1-3, dadurch gekennzeichnet, dass er zwischen den Vergrösserungswechsler (7) und den Okulartubus (8) des Mikroskops einbaubar ist.

5. Photozusatz nach einem der Ansprüche 1-4, dadurch gekennzeichnet, dass die Blende(n) (44-49) zusätzlich unabhängig vom Spiegel (22) betätigbar ist (sind).

6. Photozusatz nach einem der Ansprüche 1-5, dadurch gekennzeichnet, dass wahlweise anbaubare Objektivtuben (13), z.B. für Kleinbild- oder Sofortbild- sowie Mono- oder Stereoaufnahmen vorgesehen sind.

7. Photozusatz nach einem der Ansprüche 1-6, dadurch gekennzeichnet, dass der Spiegel (22) elektromechanisch, z.B. mittels eines Elektromagneten (104, 105) betätigt wird, wobei ein gemeinsames Betätigungselement (102) zur elektrischen Steuerung des Spiegels, der Kamera und einer eventuellen Blitzleuchte vorhanden ist.

8. Photozusatz nach Anspruch 7, dadurch gekennzeichnet, dass bezogen auf die Ansteuerung der Spiegelschwenkung die Auslösung der Kamera und der eventuellen Blitzleuchte elektrisch verzögert oder durch einen bei wirksamer Stellung des Spiegels ansprechenden Ueberwachungsschalter erfolgt.

9. Photozusatz nach einem der Ansprüche 1-8, gekennzeichnet durch zwei schwenkbare Blendenscheiben (49) mit je zwei gleichartigen, etwa halbkreisförmigen Fenstern (49a), die in entgegengesetzter Richtung in einem Bereich schwenkbar sind, in welchem sie gemeinsam im wesentlichen symmetrisch zu den optischen Achsen der binokularen Strahlengänge liegende Oeffnungen (51, 52) bilden.

10. Photozusatz nach Anspruch 9, dadurch gekennzeichnet, dass mindestens eine Rückstellfeder (46) die beiden Blendenscheiben (49) in einer durch Anschlag (48) bestimmten Endstellung hält, und dass ein gemeinsames, auf je einen Mitnehmer (44) jeder Blendenscheibe (49) wirkendes Betätigungsglied vorgesehen ist.

11. Photozusatz nach Anspruch 9 oder 10, dadurch gekennzeichnet, dass die etwa halbkreisförmigen Blendenfenster (49a) an einem Ende des gestreckten Teils auswärts erweitert sind, wobei die damit gebildeten, auswärts gezogenen Fensterecken die kleinsten Blendenöffnungen bestimmen.

## Claims

1. A photo supplement for a microscope, more particularly for an ophtalmologic microscope, for the observation of an eye while it is being illuminated by means of a slit lamp, with a photographic camera (14), comprising a totally reflecting mirror (22) which is capable of being moved into the path of rays, and at least one adjustable diaphragm (44 - 49) on the side of light incidence and in front of the mirror (22), characterised by a housing (10) which is capable of being inserted in front of the ocular tube (8) of the microscope and in which said mirror (22) and said diaphragm(s) are accommodated, a photographic tube (13) being connected to said housing which holds the camera (14) in such a manner that the actual photographic exposure is made through the diaphragm(s) (44 - 49), via the mirror (22) and through the photographic tube (13), common means (35 105) for a simultaneous displacement of the mirror (22) and of the diaphragm(s) (44- 49) from a respective resting position for observation to a position for exposure as well as an adjustable abutment (60) for the preselection of the diaphragm position for the exposure being provided.

2. Photo supplement according to claim 1, characterised in that said common means are an actuating member (35, 105) which actuates the mirror (22), e.g. by means of a spring (36, 106), the positions of the mirror being determined by abutments (41, 41a).

3. Photo supplement according to claim 2, characterised in that an actuating lever (17) acts upon the release of the camera in a delayed manner.

4. Photo supplement according to any one of claims 1 - 3, characterised in that it is capable of being mounted between the magnification selector (7) and the ocular tube (8) of the microscope.

5. Photo supplement according to any one of claims 1 - 4, characterised in that the diaphragm(s) (44-49) is (are) additionally actuatable independently from the mirror (22).

6. Photo supplement according to any one of claims 1 - 5, characterised in that selectively mountable lens cones (13) e.g. for miniature or instant pictures as well as mono or stereo exposures are provided.

7. Photo supplement according to any one of claims 1 - 6, characterised in that the mirror (22) is actuated by electromechanical means, e.g. by means of an electromagnet (104, 105), a common actuating element (102) for the electric control of the mirror, of the camera, and of a possible flashlight being provided.

8. Photo supplement according to claim 7, characterised in that the release of the camera and of the possible flashlight is electrically delayed with respect to the activation of the mirror movement or is effected by a detector switch which responds in the active position of the mirror.

9. Photo supplement according to any one of claims 1 - 8, characterised by two pivotable diaphragm disks (49) having two approximately semicircular windows (49a) of the same kind each and which are pivotable in opposite directions in a range in which they jointly form openings (51, 52) which are substantially symmetrical with respect to the optical axes of the binocular paths of rays.

10. Photo supplement according to claim 9, characterised in that at least one return spring (46) maintains the two diaphragm disks (49) in an end position which is determined by an abutment (48), and in that a common actuating member is provided which acts upon a respective catch (44) of each diaphragm disk (49).

11. Photo supplement according to claim 9 or 10, characterised in that the approximately semicircular diaphragm windows (49a) are outwardly widened at one end of their rectilinear portions, the thus formed drawn-out window corners determining the smallest diaphragm apertures.

## Revendications

1. Supplément photographique pour un microscope, plus particulièrement un microscope ophtalmologique, pour l'observation d'un oeil illuminé à l'aide d'une lampe à fente au moyen d'une caméra photographique (14), comprenant un miroir à réflexion totale (22) capable d'être placé dans la marche des rayons et au moins un diaphragme ajustable (44 - 49) sur le côté d'incidence de la lumière et devant le miroir (22), caractérisé par un boîtier (10) pouvant être inséré devant le tube oculaire (8) du microscope, dans lequel se trouvent ledit miroir (22) et le (les) diaphragme(s) et auquel boîtier est relié un tube photographique (13) qui porte une caméra (14) de telle manière que les expositions photographiques sont prises par l'intermédiaire du (des) diaphragme(s) (44 - 49), du miroir (22) et du tube photographique (13), des moyens communs (35, 105) pour le déplacement simultané du miroir (22) et du (des) diaphragme(s) (44- 49) d'une position de repos pour l'observation à une position pour l'exposition ainsi qu'un élément d'arrêt (60) ajustable pour la présélection de la position de diaphragme pour l'exposition étant prévus.

2. Supplément photographique selon la revendication 1, caractérisé en ce que lesdits moyens communs sont constitués par un membre d'actionnement (35, 105) qui commande, par exemple au moyen d'un ressort (36, 106), le miroir (22) dont les positions sont déterminés par des arrêts (41, 41a).

3. Supplément photographique selon la revendication 2, caractérisé en ce qu'un levier d'actionnement (17) agit sur le déclenchement de la caméra de manière retardée.

4. Supplément photographique selon l'une quelconque des revendications 1 à 3, caractérisé en ce qu'il est capable d'être monté entre le changeur de grossissement (7) et le tube oculaire (8) du microscope.

5. Supplément photographique selon l'une quelconque des revendications 1 à 4, caractérisé en ce que le (les) diaphragme(s) (44 - 49) est (sont) additionnellement capables d'être actionné(s) indépendamment du miroir (22).

6. Supplément photographique selon l'une quelconque des revendications 1 à 5, caractérisé en ce que des tubes d'objectif (13) séléctivement attachables, par exemple pour des prises de petit format ou instantanées ainsi que pour des expositions monoculaires ou stéréoscopiques, sont prévus.

7. Supplément photographique selon l'une quelconque des revendications 1 à 6, caractérisé en ce que le miroir (22) est actionné par voie électromagnétique, par exemple au moyen d'un électro-aimant (104, 105), un élément d'actionnement (102) commun pour la commande électrique du miroir, de la caméra et d'un flash éventuel étant pourvu.

8. Supplément photographique selon la revendication 7, caractérisé en ce que le déclenchement de la caméra et du flash éventuel est électriquement retardé par rapport à l'actionnement du mouvement pivotant du miroir ou effectué par un interrupteur de détection répondant à la position effective du miroir.

9. Supplément photographique selon l'une quelconque des revendications 1 à 8, caractérisé par deux diaphragmes (49) en forme de disque présentant chacun deux fenêtres (49a) approximativement semicirculaires du même type et pouvant être pivotés en directions opposées dans un domaine dans lequel ils forment ensemble des ouvertures (51, 52) essentiellement symétriques aux axes optiques des marches des rayons binoculaires.

10. Supplément photographique selon la revendication 9, caractérisé en ce qu'au moins un ressort de retour (46) maintient les deux diaphragmes en forme de disque (49) dans une position extrême déterminée par un arrêt (48), et en ce qu'un membre d'actionnement commun agissant sur un élément d'entraînement (44) correspondant de chaque diaphragme en forme de disque (49) est prévu.

11. Supplément photographique selon la revendication 9 ou 10, caractérisé en ce que les fenêtres (49a) approximativement semicirculaires des diaphragmes sont élargies vers l'extérieur à l'une des extrémités de leurs parties allongées, les coins étendus vers l'extérieur des fenêtres ainsi formés déterminant les ouvertures de diaphragme minimales.
